# EUROPEAN PATENT SPECIFICATION

(11) **EP 4 277 628 B1**
(45) Date of publication and mention of the grant of the patent: **24.09.2025**
(21) Application number: 22700096.5
(22) Date of filing: 17.01.2022
(51) Int. Cl.: A61K 31/4704, A61K 38/17, A61K 35/00, A61N 5/00, A61K 9/00, A61P 35/00, A61P 35/02

(54) **TASQUINIMOD OR A PHARMACEUTICALLY ACCEPTABLE SALT THEREOF FOR USE IN THE TREATMENT OF MYELODYSPLASTIC SYNDROME**
TASQUINIMOD ODER PHARMAZEUTISCH UNBEDENKLICHES SALZ DAVON ZUR VERWENDUNG BEI DER BEHANDLUNG DES MYELODYSPLASTISCHEN SYNDROMS
TASQUINOMOD OU SEL PHARMACEUTIQUEMENT ACCEPTABLE DE CELUI-CI DESTINÉ À ÊTRE UTILISÉ DANS LE TRAITEMENT DU SYNDROME MYÉLODYSPLASIQUE

(30) Priority: 18.01.2021 EP 21152018; 07.10.2021 EP 21201509; 29.10.2021 EP 21205665
(43) Date of publication of application: 22.11.2023
(73) Proprietor: Active Biotech AB, 223 63 Lund (SE)
(72) Inventor: WOBUS, Manja, 01307 DRESDEN (DE); SOCKEL, Katja, 01307 DRESDEN (DE); BORNHÄUSER, Martin, 01307 DRESDEN (DE)
(74) Representative: Brann AB
(86) International application number: PCT/EP2022/050891
(87) International publication number: WO 2022/152902

(56) References cited:
- WO-A1-2004/035064
- WO-A1-2016/078921
- ULRIKE BACHER ET AL: "Recent advances in diagnosis, molecular pathology and therapy of chronic myelomonocytic leukaemia", BRITISH JOURNAL OF HAEMATOLOGY, JOHN WILEY, HOBOKEN, USA, vol. 153, no. 2, 9 March 2011 (2011-03-09), pages 149 - 167, XP071161672, ISSN: 0007-1048, DOI: 10.1111/J.1365-2141.2011.08631.X
- RAYMOND E ET AL: "Mechanisms of action of tasquinimod on the tumour microenvironment", CANCER CHEMOTHERAPY AND PHARMACOLOGY, SPRINGER VERLAG , BERLIN, DE, vol. 73, no. 1, 27 October 2013 (2013-10-27), pages 1 - 8, XP035339581, ISSN: 0344-5704, [retrieved on 20131027], DOI: 10.1007/S00280-013-2321-8
- ANONYMOUS: "Myelodysplastic Syndromes Treatment (PDQ )-Health Professional Version - National Cancer Institute", 17 June 2021 (2021-06-17), XP055907558, Retrieved from the Internet <URL:https://www.cancer.gov/types/myeloproliferative/hp/myelodysplastic-treatment-pdq> [retrieved on 20220331]

## Description

### FIELD OF THE INVENTION

The present invention relates to a novel use of the quinoline derivative tasquinimod. More particularly, the invention relates to tasquinimod or a pharmaceutically acceptable salt thereof for use in the treatment of myelodysplastic syndrome.

### BACKGROUND OF THE INVENTION

Tasquinimod and a method for its preparation were described in International Applications No. PCT/SE99/00676, published as WO 99/55678, and No. PCT/SE99/01270, published as WO 00/03991, which applications also disclosed the utility of tasquinimod and some other quinoline carboxamides for the treatment of diseases resulting from autoimmunity, such as multiple sclerosis, insulin-dependent diabetes mellitus, systemic lupus erythematosus, rheumatoid arthritis, inflammatory bowel disease and psoriasis and, furthermore, diseases where pathologic inflammation plays a major role, such as asthma, atherosclerosis, stroke and Alzheimer's disease.

Processes for preparing tasquinimod are also disclosed in International Application No. PCT/SE2003/000780, published as WO 03/106424 and International Application No. PCT/EP2011/061490, published as WO 2012/004338. A deuterated form of tasquinimod was described in International Application No. PCT/EP2012/061798, published as WO 2012/175541.

The use of various quinoline carboxamides for the treatment of cancer, more particularly solid cancers, such as prostate cancer and breast cancer, is disclosed in International Application No. PCT/SE00/02055, published as WO 01/30758. It has been found that some of these compounds bind to and inhibit the interactions of an immunomodulatory protein (S100A9), which protein promotes tumor development, influences suppressive and pro-angiogenic cells in the tumor microenvironment and participates in the establishment of pre-metastatic niches.

International Application No. PCT/EP2015/075769, published as WO 2016/078921, discloses tasquinimod for use in the treatment of leukemia including acute lymphoblastic leukemia, acute myeloid leukemia, chronic lymphocytic leukemia and chronic myeloid leukemia.

International Application No. PCT/EP2015/071391, published as WO 2016/042112, discloses tasquinimod for use in the treatment of multiple myeloma.

International Application No. PCT/EP2016/053288, published as WO 2016/146329, discloses tasquinimod for use in combination with a PD-1 and/or PD-L1 inhibitor in the treatment of cancer, in particular bladder cancer.

International Application No. PCT/US2003/011323, published as WO 2004/035064, discloses immunomodulatory compounds for use in the treatment and management of MDS. The immunomodulatory compounds are defined as small organic molecules that markedly inhibit TNF-α, LPS induced monocyte IL1β and IL12, and partially inhibit IL6 production, and that additionally enhance the degradation of TNF-α mRNA.

Myelodysplastic syndromes (MDS) are a heterogeneous spectrum of chronic myeloid neoplasia or clonal hematopoietic stem cell disorders, associated with ineffective hematopoiesis manifesting as symptomatic cytopenias, morphologic dysplasia and a substantial risk of progression to acute myeloid leukemia.

MDS are one of the most common haematological neoplasms in the elderly population with a median age of 65-70 years; however, disease can occur at any age. While no apparent risk factor is present in primary MDS, DNA damage by previous chemo- or radiation therapy is a well-known risk factor for secondary MDS. Further, MDS in the paediatric population might arise secondary to inherited bone marrow failures (for example Fanconi anemia, or dysceratosis congenital).

Multiple biological processes govern hematopoietic progenitor proliferation and differentiation into mature blood cells. Molecular alterations in hematopoietic stem cells that disrupt any of these processes are integral to the pathogenesis of MDS. About 80-90% of MDS patients have a somatic mutation in their hematopoietic stem cells. The type and incidence of somatic mutations contribute to dysfunctional signalling pathways in MDS and are associated with disease prognosis and responsiveness to certain drugs. Besides molecular alterations of the hematopoietic stem cell, the surrounding bone marrow niche plays a central role in MDS pathogenesis. Aberrant inflammatory signalling, including the activation of the NLRP3 inflammasome, may facilitate the selection, maintenance and leukemic evolution of the malignant MDS clone.

MDS patients can be asymptomatic at diagnosis, however, up to 80% suffer from anemia. Patients are primarily affected by cytopenia-related symptoms, such as fatigue and weakness due to anemia, neutropenia-related infections or bleeding due to thrombocytopenia. About 1/3 of the MDS patients will progress to acute myeloid leukemia.

The revised International Prognostic Scoring System for MDS (IPSS-R) is the most common used prognostication system to determine disease prognosis. The model demonstrates 5 risk categories according to the grade of cytopenia, marrow blast percentage and cytogenetic subgroup.

Even though MDS is one of the most common hematologic neoplasms in the elderly population, only limited treatment options are available. Besides supportive care, only five drugs are approved in Europe (erythropoietin, luspatercept, the iron chelator deferasirox, the immunomodulatory drug lenalidomide, and the hypomethylating agent azacitidine). The only curative treatment is allogeneic hematopoietic stem cell transplantation, which is however not feasible in many elderly patients.

Supportive care includes transfusion therapy, treatment with erythropoiesis-stimulating agents, and antibiotic therapy. Transfusion therapy (blood transfusion) is a method of giving red blood cells, white blood cells, or platelets to replace blood cells destroyed by disease or treatment. Frequent red blood cell transfusions lead to iron overload and accompanying organ toxicities with poor clinical outcome. Iron chelation therapy with deferasirox might improve patient's outcome. Erythropoiesis-stimulating agents (ESAs) are given to increase the number of mature red blood cells made by the body and to lessen the effects of anemia. Sometimes granulocyte colony-stimulating factor (G-CSF) is given with ESAs to help the treatment work better. Finally, antibiotic therapy may be used to fight infection.

Drug therapy includes treatment with e.g. lenalidomide, which is only approved in patients with myelodysplastic syndrome associated with an isolated del(5q) chromosome abnormality who need frequent red blood cell transfusions. Patients with transfusion-dependent MDS with ring sideroblasts, refractory or unlikely to respond to ESA, can use luspatercept. The hypomethylating agent azacitidine may be used to treat myelodysplastic syndromes in advanced disease stages to slow the progression of myelodysplastic syndromes to acute myeloid leukemia. Usually, classical chemotherapy is only used before stem cell transplantation in patients with higher blast count to reduce tumor cell burden.

During allogeneic stem cell transplantation, stem cells (immature blood cells) are collected from the blood or bone marrow of a healthy donor and reinfused to the MDS patient after completion of conditioning therapy. The donor hematopoietic stem cells will replace the patient's original bone marrow and restore the body's blood cells (termed engraftment).

### SUMMARY OF THE INVENTION

A first aspect is tasquinimod or a pharmaceutically acceptable salt thereof for use in the treatment of myelodysplastic syndrome.

A further aspect is a pharmaceutical formulation containing a therapeutically effective amount of tasquinimod or a pharmaceutically acceptable salt thereof, for the treatment of myelodysplastic syndrome.

Further aspects and embodiments thereof will become apparent from the following description and claims.

### BRIEF DESCRIPTION OF THE DRAWINGS

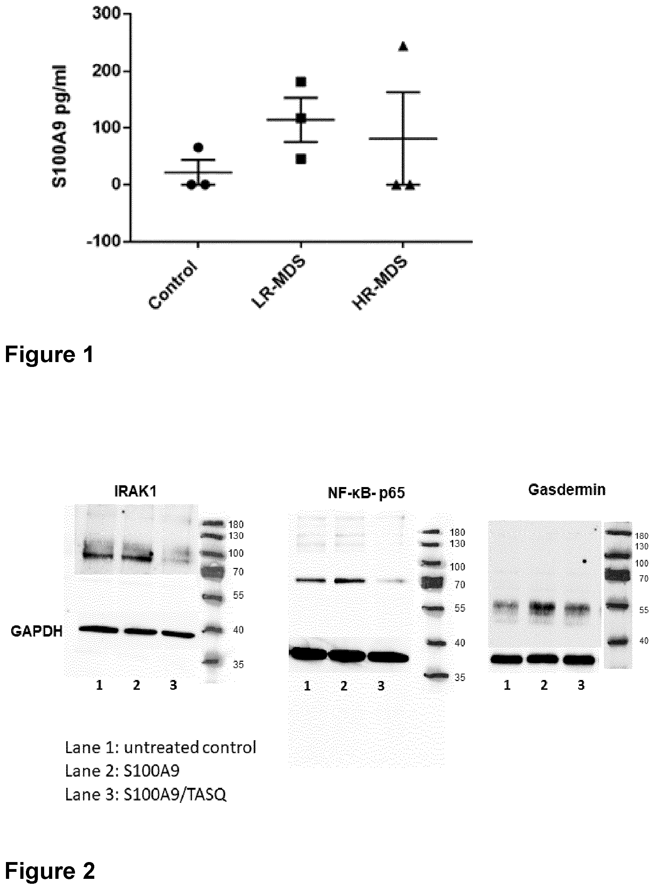

FIGURE 1 is a graph representing the concentration of S100A9 (in pg/ml) in bone marrow obtained from healthy patients (control), and patients with low risk MDS (LR-MDS) and high risk MDS (HR-MDS), respectively.
FIGURE 2 shows Western blots of phosphorylated IRAK1 (80 kDa), NF-κB-p65 (65 kDa) and gasdermin (50 kDa) in MDS MSCs following treatment with S100A9 or with S100A9 and tasquinimod, for 48h. GAPDH (38 kDa) served as reference protein. Lane 1 = untreated control; lane 2 = treated with S100A9; lane 3 = treated with S100A9/tasquinimod.

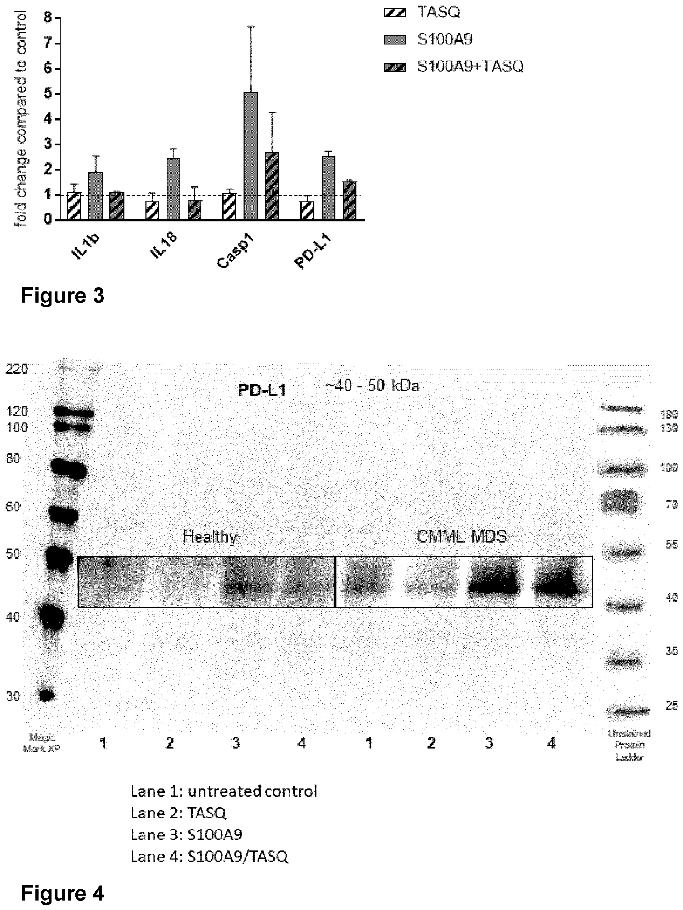

FIGURE 3 is a bar chart representing the mRNA expression levels of IL-1β, IL-18, Casp1 and PD-L1 by real-time PCR of MDS MSCs after 48h treatment with tasquinimod only (TASQ), with S100A9 only (S100A9), or with both S100A9 and tasquinimod (S100A9+TASQ). Amplicons for IL-1β, IL-18, Casp1 and PD-L1 were normalized to endogenous GAPDH expression and the untreated MSCs were set to 1 (=control).
FIGURE 4 shows a Western blot of PD-L1 in healthy and CMML MSCs following treatment with S100A9 and tasquinimod for 48h. Lane 1 = untreated control; lane 2 = treated with tasquinimod; lane 3 = treated with S100A9; lane 3 = treated with S100A9 and tasquinimod.

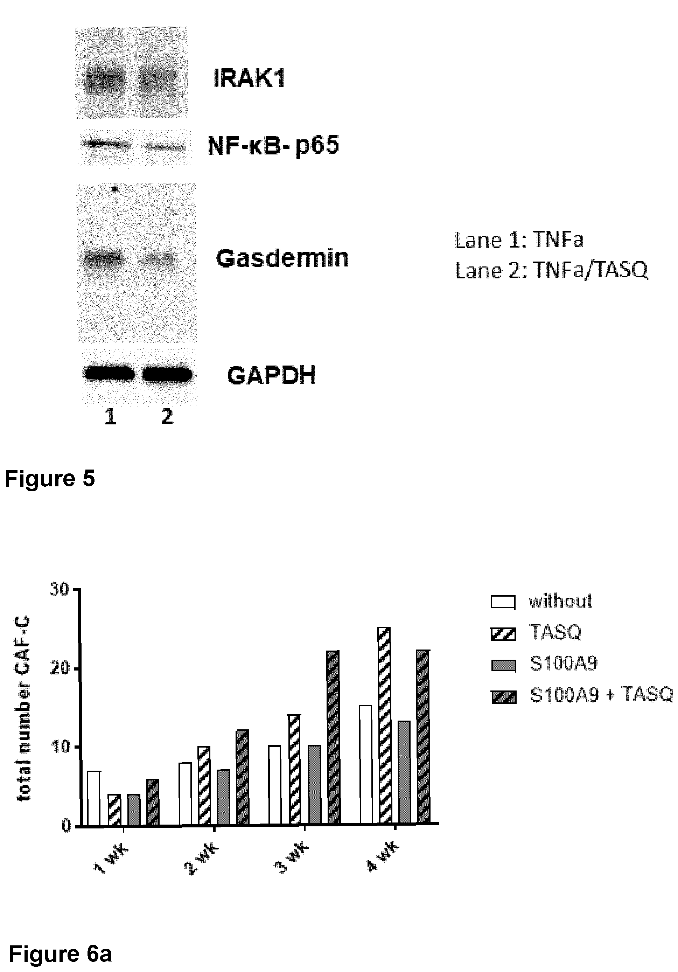

FIGURE 5 shows a Western blot of phosphorylated IRAK1 (80 kDa), NF-κB-p65 (65 kDa) and gasdermin (50 kDa) in MDS MSCs following treatment with TNF-α and tasquinimod for 48h. GAPDH (38 kDa) served as reference protein. Lane 1 = treated with TNF-α; lane 2 = treated with TNF-α and tasquinimod.
FIGURE 6a is a bar chart showing quantification of CAF-C when co-culturing MDS MSC with hematopoietic progenitor cells, after one, two, three and four weeks without the presence of either S100A9 or tasquinimod ("without"), in the presence of tasquinimod (TASQ), in the presence of S100A9 (S100A9), and in the presence of both s100A9 and tasquinimod (S100A9+TASQ).

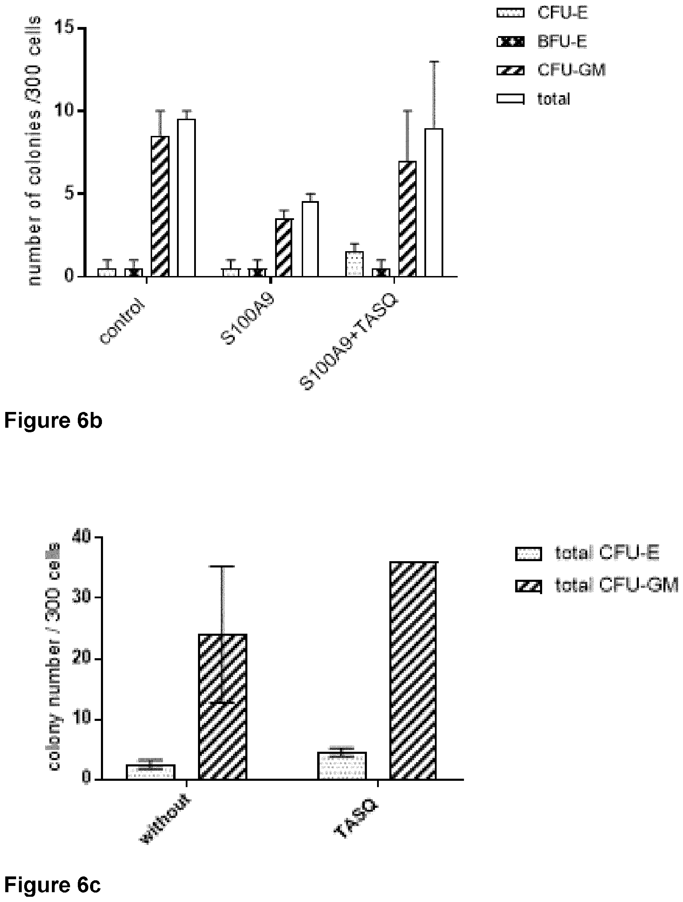

FIGURE 6b is a bar chart showing the number of colonies/300 cells in CFU assays performed using MDS MSC cells cocultured with hematopoietic progenitor cells, harvested after one week of co-culture in the presence of S100A9 or in the presence of both S100A9 and tasquinimod or in the absence of both S100A9 and tasquinimod ("control"), with 300 cells being plated in enriched methylcellulose medium with recombinant cytokines (MethoCult^{™} H4435, STEMCELL Technologies). Colonies were counted after 2 weeks and classified under a microscope or with the STEMvision^{™} system (STEMCELL Technologies).
FIGURE 6c is a bar chart showing the number of colonies/300 cells in CFU assays performed using MDS MSC cells and hematopoietic progenitor cells, harvested after one week of co-culture in the presence ("TASQ") or absence ("without") of tasquinimod, with 300 cells being plated in enriched methylcellulose medium with recombinant cytokines (MethoCult^{™} H4435, STEMCELL Technologies). Colonies were counted after 2 weeks and classified under a microscope or with the STEMvision^{™} system (STEMCELL Technologies).

### DETAILED DESCRIPTION

Unless otherwise defined, all technical and scientific terms used herein have the same meaning as commonly understood by one of ordinary skill in the art to which this invention pertains.

The abbreviations and acronyms used in this text are considered as well-known to the person of ordinary skill in the field art, but for the sake of clarity the meaning of some of these is indicated herein below:
- ALP: alkaline phosphatase
- αSMA: alpha-smooth muscle actin
- BCA: bicinchoninic acid assay
- BFU-E: burst forming unit-erythroid
- BM: bone marrow
- CAF-C: cobblestone area forming-cell
- cDNA: complementary DNA
- CFU: colony forming unit
- CFU-E: colony forming unit-erythroid
- CFU-GEMM: colony forming unit-granulocyte, erythrocyte, monocyte, megakaryocyte
- CFU-GM: colony forming unit-granulocyte/macrophage
- CMML: chronic myelomonocytic leukemia
- Cy: cyanine
- DAPI: 4',6-diamidino-2-phenylindole
- DMEM: Dulbecco's Modified Eagle Medium
- dT: deoxythymidine
- ECAR: extracellular acidification rate
- ECL: enhanced chemiluminescence
- EDTA: ethylenediaminetetraacetic acid
- ELISA: enzyme-linked immunosorbent assay
- FCCP: carbonyl cyanide-p-trifluoromethoxyphenylhydrazone
- FCS: fetal calf serum
- FLT3-L: fms-like tyrosine kinase 3 ligand
- GAPDH: glyceraldehyde 3-phosphate dehydrogenase
- HRP: horseradish peroxidase
- HSA: human serum albumin
- HSC(s): hematopoietic stem cell(s)
- IgG: immunoglobulin G
- IL: interleukin
- IRAK1: Interleukin-1 receptor-associated kinase 1
- MDS: myelodysplastic syndrome
- MDS-RS: myelodysplastic syndrome with ring sideroblasts
- MSC(s): mesenchymal stem cell(s)
- NF-κB: nuclear factor kappa-light-chain-enhancer of activated B cells
- OCR: oxygen consumption rate
- PBMC: Peripheral blood mononuclear cell
- PBS: phosphate buffered saline
- PD-1: programmed cell death protein 1
- PD-L1: programmed death-ligand 1
- RIPA: radioimmunoprecipitation assay
- RNA: ribonucleic acid
- ROS: reactive oxygen species
- ROX: carboxy-X-rhodamine
- RT-PCR: real-time polymerase chain reaction
- SCF: stem cell factor
- SDS: sodium dodecyl sulfate
- TLR4: toll like receptor 4
- TNF-α: tumor necrosis factor alpha

The compound tasquinimod, or 4-hydroxy-5-methoxy-N,1-dimethyl-2-oxo-N-[4-(trifluoromethyl)phenyl]-1,2-dihydroquinoline-3-carboxamide, has the structural formula

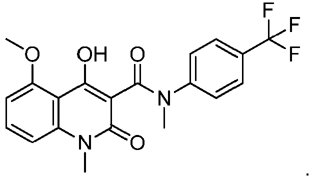

Optional" or "optionally" means that the subsequently described event or circumstance may but need not occur, and that the description includes instances where the event or circumstance occurs and instances in which it does not.

"Pharmaceutically acceptable" means that which is useful in preparing a pharmaceutical composition that is generally safe, non-toxic, and neither biologically nor otherwise undesirable and includes that which is acceptable for veterinary use as well as human pharmaceutical use.

Examples of pharmaceutically acceptable salts comprise salts with (as counter ion) an alkali metal ion, e.g. Li⁺, Na⁺ or K⁺, or salts with an alkaline earth metal ion, e.g. Mg²⁺ or Ca²⁺, or salts with any other pharmaceutically acceptable metal ion, e.g. Zn²⁺ or Al³⁺; or pharmaceutically acceptable salts formed with organic bases, such as diethanolamine, ethanolamine, N-methylglucamine, triethanolamine or tromethamine.

"Therapeutically effective amount" means an amount of tasquinimod or a pharmaceutically salt thereof, that, when administered to a subject for treating a condition (here: MDS), is sufficient to effect such treatment for the condition. The "therapeutically effective amount" will vary depending on e.g. the age and relative health of the treated subject, the state of progression of the condition, the route and form of administration, the possible additional use of other drugs, e.g. in a combination therapy, etc. In some embodiments, an "effective amount" provides treatment effect (e.g., treats, prevents, inhibits, relieves, promotes, improves, increases, reduces, and the like) as measured by a statistically significant change in one or more indications, symptoms, signs, diagnostic tests, vital signs, and the like. In further embodiments, an "effective amount" suppresses, manages, or prevents a condition as measured by a lack of a statistically significant change in one or more indications, symptoms, signs, diagnostic tests, vital signs, and the like.

As used herein the terms "treatment" or "treating" is an approach for obtaining beneficial or desired results including clinical results. Beneficial or desired clinical results can include, but are not limited to, alleviation or amelioration of one or more symptoms of the treated condition, diminishment of extent of the condition, stabilization (i.e., not worsening) of the state of the condition, preventing spread of the condition, delay or slowing of progression of the condition, amelioration or palliation of the condition, and remission (whether partial or total), whether detectable or undetectable. The term can also mean prolonging survival as compared to expected survival without the treatment.

MDS patients are primarily affected by cytopenia, such as anemia, thrombocytopenia, neutropenia, bicytopenia and pancytopenia and their related symptoms, such as fatigue, anemia, weakness, easy bruising or bleeding, fever, bone pain, shortness of breath, and frequent infections.

The term "mammal" refers to a human or any mammalian animal, e.g. a primate, a farm animal, a pet animal, or a laboratory animal. Preferably, the mammal is a human.

The mammal (e.g. human) subject that may suitably be treated according to the present invention may be one suffering from MDS, or one at (increased) risk of developing MDS.

As used herein, "MDS" refers to a group of acquired hematopoietic disorders characterized by peripheral cytopenias and a normocellular or hypercellular bone marrow. Subtypes of MDS include MDS with unilineage (single lineage) dysplasia, MDS with multilineage dysplasia, MDS with ring sideroblasts (MDS-RS), MDS associated with isolated del chromosome abnormality, such as MDS with isolated del(5q), MDS with excess blasts, and MDS, unclassifiable.

The WHO has classified the various forms of MDS into different subtypes, depending on criteria such as the percentage of myeloblasts in the bone marrow, the presence of abnormal red blood cell precursors (ringed sideroblasts) in the bone marrow, the number of abnormal cell types known as dysplastic lineages in the bone marrow, and the genetic profile of the bone marrow cells, as follows:
MDS with single-lineage dysplasia (MDS-SLD) - 1 or 2 blood cytopenias; in bone marrow, dysplasia in ≥ 10% of one cell line, < 5% blasts
MDS with multilineage dysplasia (MDS-MLD) - 1-3 blood cytopenias, < 1 × 10⁹/L monocytes; in bone marrow, dysplasia in ≥ 10% of cells in ≥ 2 hematopoietic lineages < 15% ring sideroblasts (or < 5% ring sideroblasts if *SF3B1* mutation present) < 5% blasts
MDS with ring sideroblasts (MDS-RS) - anemia, no blasts; in bone marrow, ≥ 15% of erythroid precursors with ring sideroblasts or ≥ 5% ring sideroblasts if *SF3B1* mutation is present
MDS with isolated del(5q) - anemia, platelets normal or decreased; in bone marrow, unilineage erythroid dysplasia, isolated del(5q), < 5% blasts ± one other abnormality except - 7/del(7q)
MDS with excess blasts (MDS-EB) - 1-3 blood cytopenias, 0-3 dysplastic bone marrow lineages, and 5-9% blasts in bone marrow or 2-4% blasts in blood (MDS-EB1) or 10-19% blasts in bone marrow or 5-19% blasts in blood (MDS-EB2)

Unclassifiable MDS - cytopenias, ±1% blasts on at least 2 occcasions; in bone marrow, single-lineage dysplasia or no dysplasia but characteristic MDS cytogenetics, < 5% blasts.

The WHO classification also includes the provisional category of refractory cytopenia of childhood, with cytopenias and < 2% blasts in peripheral blood and, in bone marrow, dysplasia in 1-3 lineages and < 5% blasts.

Additionally, according to the WHO classification, CMML (CMML-0, CMML-1, CMML-2 ) and MDS/MPN-RS-T are classical MDS/MPN overlap syndromes.

The classification developed by the WHO is used herein to define the various subtypes of MDS, but unless otherwise specified or apparent from the context, the term MDS as used herein is considered to include any subtype of MDS, e.g. any of the above indicated subtypes, also including CMML. In some embodiments, however, the MDS more particularly is MDS with single-lineage dysplasia as defined herein above. In some embodiments, the MDS more particularly is MDS with multilineage dysplasia (MDS-MLD) as defined herein above. In some embodiments, the MDS more particularly is MDS with ring sideroblasts (MDS-RS) as defined herein above. In some embodiments, the MDS more particularly is MDS with isolated del(5q) as defined herein above. In some embodiments, the MDS more particularly is MDS with excess blasts (MDS-EB) as defined herein above. In some embodiments, the MDS more particularly is unclassifiable MDS as defined herein above. In some embodiments, the MDS more particularly is refractory cytopenia of childhood, as defined herein above. In some embodiments, the MDS more particularly includes CMML. In some further embodiments, the MDS does not include CMML.

In addition to the diagnostic classification system mentioned above, a prognostic system, called Revised International Prognostic Scoring System (IPSS-R) has been developed, according to which MDS can be classified as very low risk, low risk, intermediate risk or high risk or very high risk, cf. guidance published by Greenberg, Tuechler, Schanz et al., Revised International Prognostic Scoring System (IPSS-R for Myelodysplastic Syndrome, Blood 120: 2454, 2012), and Schanz J et al, (J Clin Oncology 2012; 30:820). The system uses hemoglobin count (g/dl), absolute neutrophil count (x10⁹/L), platelets (x10⁹/L), bone marrow blast (percent), and cytogenetic category of the MDS. The cytogenetic category is defined as very good, good, intermediate, poor, or very poor, based on the presence of certain cytogenetic abnormalities, as represented in Table 1.

**Table 1**

| CYTOGENETIC PROGNOSTIC SUBGROUPS | CYTOGENETIC ABNORMALITIES |
|---|---|
| Very good | -Y, del(11q) |
| Good | Normal, del(5q), del(12p), del(20q), double including del(5q) |
| Intermediate | del(7q), +8, +19, i(17q), any other single or double independent clones |
| Poor | -7, inv(3)/t(3q)/del(3q), double including -7/del(7q), Complex: 3 abnormalities |
| Very poor | Complex: >3 abnormalities |

The cytogenetic prognostic subgroup, and the determined value of each of the above-mentioned criteria (hemoglobin count (g/dl), absolute neutrophil count (ANC) (x10⁹/L), platelets (x10⁹/L), bone marrow blast (percent)) of the patient are scored as shown in Table 2

**Table 2**

| PROGNOSTIC VARIABLE | 0 | 0.5 | 1 | 1.5 | 2 | 3 | 4 |
|---|---|---|---|---|---|---|---|
| Cytogenetics | Very Good | | Good | | Intermediate | Poor | Very Poor |
| BM Blast (%) | ≤2 | | >2-<5% | | 5-10% | >10% | |
| Hemoglobin (g/dl) | ≥10 | | 8-<10 | <8 | | | |
| Platelets x10⁹/L | ≥100 | 50-<100 | <50 | | | | |
| ANC x10⁹/L | ≥0.8 | <0.8 | | | | | |

The total risk score obtained is used to classify the MDS into an IPSS-R Prognostic Risk Category, as shown in Table 3

**Table 3**

| RISK CATEGORY | RISK SCORE |
|---|---|
| Very Low | ≤1.5 |
| Low | >1.5 - 3 |
| Intermediate | >3 - 4.5 |
| High | >4.5 - 6 |
| Very High | >6 |

Finally, based on data from over 7000 patients, the IPSS-R has associated each prognostic risk category with a median survival time in years and a median time to 25 % AML evolution, as indicated in Table 4.

**Table 4**

| Risk category | Very Low | Low | Intermediate | High | Very High |
|---|---|---|---|---|---|
| Survival* | 8.8 years | 5.3 years | 3.0 years | 1.6 years | 0.8 year |
| AML/25 %** | NR | 10.8 years | 3.2 years | 1.4 years | 0.7 year |

| | | | | | |
|---|---|---|---|---|---|
| * medians, ** median time to 25 % AML evolution | | | | | |

In some embodiments, the indication MDS includes various forms of anemia, such as refractory anemia, refractory anemia with ringed sideroblasts, refractory anemia with excess blasts, refractory anemia with excess blasts in transformation and chronic myelomonocytic leukemia. In some embodiments, the indication MDS excludes MDS that has developed into leukemia. In some embodiments, the patient does not have a leukemia.

Tasquinimod may be used in the treatment of an MDS that has been classified into a risk score according to the Revised International Prognostic Scoring System (IPSS-R) for Myelodysplastic Syndromes as mentioned herein.

Unless otherwise specified herein, the MDS may be within any of the above identified risk categories, as determined by use of the above-mentioned Revised International Prognostic Scoring System (IPSS-R).

The MDS may belong to the risk category defined as very low (having a risk score of lower than or equal to 1.5), low (having a risk score of higher than 1.5 and lower than or equal to 3), or intermediate (having a risk score of higher than 3 and lower than or equal to 4.5). In some of these embodiments, the MDS belongs to the risk category defined as low or intermediate, e.g. as intermediate. In some further embodiments, the MDS belongs to the risk category defined as very low or low, e.g. as low. In still further embodiments, the MDS belongs to the risk category defined as very low.

The MDS may belong to the risk category defined as intermediate, high (having a risk score of higher than 4.5 and lower than or equal to 6) or very high (having a risk score of higher than 6). For example, the MDS may belong to the risk category defined as intermediate or high, e.g. as high, or may belong to the risk category defined as high or very high, e.g. as very high.

Anemia is an important, not to say predominant, cause of morbidity and quality of life impairment in MDS patients, in particular those with lower risk MDS (e.g., very low risk, low risk, or intermediate risk MDS). In some embodiments, tasquinimod is used in the treatment of MDS associated with anemia as a symptom thereof. In some embodiments, the anemia is selected from refractory anemia, refractory anemia with ringed sideroblasts, refractory anemia with excess blasts, refractory anemia with excess blasts in transformation and chronic myelomonocytic leukemia. In some embodiments, the anemia is refractory anemia. In some embodiments, the anemia is refractory anemia with ringed sideroblasts. In some embodiments, the anemia is refractory anemia with excess blasts. In some embodiments, the anemia is refractory anemia with excess blasts in transformation and chronic myelomonocytic leukemia.

In some embodiments, tasquinimod is for use in the treatment of an MDS in a patient for which at least one clinical parameter has been determined, selected from hemoglobin count, absolute neutrophil count, platelet count, bone marrow blast (percent), and cytogenetic abnormalities.

For example, in some embodiments, the MDS belongs to the cytogenetic prognostic subgroup termed "very good", as defined in the Table 1. In some other embodiments, the MDS belongs to the cytogenetic prognostic subgroup termed "good", as defined in Table 1. In still other embodiments, the MDS belongs to the cytogenetic prognostic subgroup termed "intermediate", as defined in Table 1. In still other embodiments, the MDS belongs to the cytogenetic prognostic subgroup termed "poor", as defined in Table 1. In still other embodiments, the MDS belongs to the cytogenetic prognostic subgroup termed "very poor", as defined in Table 1.

In some embodiments, the MDS patient has a BM blast of less than 2%. In some embodiments, the MDS patient has a BM blast of higher than or equal to 2% and lower than 5%. In some embodiments, the MDS patient has a BM blast of higher than or equal to 5% and lower than 10%. In some embodiments, the MDS patient has a BM blast of higher than 10%.

In some embodiments, the MDS patient has a hemoglobin level of higher than or equal to 10 g/dl. In some embodiments, the MDS patient has a hemoglobin level of higher than or equal to 8 g/dl and lower than 10 g/dl. In some embodiments, the MDS patient has a hemoglobin level of lower than 8 g/dl.

In some embodiments, the MDS patient has a platelet count of higher than or equal to 100 x 10⁹/L. In some embodiments, the MDS patient has a platelet count of higher than or equal to 50 x 10⁹/L and lower than 100 10⁹/L. In some embodiments, the MDS patient has a platelet count of lower than 50 x 10⁹/L
In some embodiments, the MDS patient has an absolute neutrophil count of higher than or equal to 0.8 x 10⁹/L. In some embodiments, the MDS patient has an absolute neutrophil count of lower than 0.8 x 10⁹/L
One or more hematologic parameters in a mammal suffering from or at risk of developing MDS (e.g. a mammal patient having MDS) may be improved by treatment with tasquinimod. Such improvement of a hematological parameter may be selected from decreasing myoblasts, increasing hemoglobin, increasing platelets, increasing neutrophils, decreasing hepcidin, reducing units of red blood cell transfused, reducing frequency of transfusion, and reducing transfusion dependence.

As mentioned herein above, tasquinimod, pharmaceutically acceptable salts thereof, crystalline salts thereof, and pharmaceutical compositions containing the compounds and their salts, as well as methods for preparing such compounds, their salts and pharmaceutical compositions containing the compounds and their salts have been described in WO 99/55678, WO 00/03991, WO 03/106424, WO 2005/074899, WO 2012/004338 and WO 2012/175541 (*vide supra*)*.*

The present invention includes tasquinimod or a pharmaceutically acceptable salt thereof, formulated in a pharmaceutical composition, optionally together with a pharmaceutically acceptable excipient, e.g. a carrier, for use in the treatment of MDS.

In some embodiments, use is made of a pharmaceutically acceptable salt of tasquinimod.

The pharmaceutical composition may be suitable for enteral administration, such as rectal or oral administration, or for parenteral administration to a mammal (especially a human), and comprises a therapeutically effective amount of tasquinimod or a pharmaceutically acceptable salt thereof, as active ingredient, optionally in association with a pharmaceutically acceptable excipient, e.g. a pharmaceutically acceptable carrier. The therapeutically effective amount of the active ingredient is as defined herein above and depends e.g. on the species of mammal, the body weight, the age, the individual condition, individual pharmacokinetic data, and the mode of administration.

For enteral, e.g. oral, administration, tasquinimod may be formulated in a wide variety of dosage forms. The pharmaceutically acceptable carriers may be either solid or liquid. Solid form preparations include powders, tablets, pills, lozenges, capsules, cachets, suppositories, and dispersible granules. A solid carrier may be one or more substances which may also act as diluents, flavouring agents, solubilizers, lubricants, suspending agents, binders, preservatives, tablet disintegrating agents, or an encapsulating material. In powders, the carrier generally is a finely divided solid which is a mixture with the finely divided active component. In tablets, the active component generally is mixed with the carrier having the necessary binding capacity in suitable proportions and compacted in the shape and size desired. Suitable carriers include but are not limited to magnesium carbonate, magnesium stearate, talc, sugar, lactose, pectin, dextrin, starch, gelatine, tragacanth, methylcellulose, sodium carboxymethylcellulose, a low melting wax, cocoa butter, and the like.

Other forms suitable for oral administration include liquid form preparations including emulsions, syrups, elixirs, aqueous solutions, aqueous suspensions, or solid form preparations which are intended to be converted shortly before use to liquid form preparations. Emulsions may be prepared in solutions, for example, in aqueous propylene glycol solutions or may contain emulsifying agents, for example, such as lecithin, sorbitan monooleate, or acacia. Aqueous solutions can be prepared by dissolving the active component in water and adding suitable colorants, flavors, stabilizers, and thickening agents. Aqueous suspensions can be prepared by dispersing the finely divided active component in water with viscous material, such as natural or synthetic gums, resins, methylcellulose, sodium carboxymethylcellulose, and other well-known suspending agents. Solid form preparations include solutions, suspensions, and emulsions, and may contain, in addition to the active component, colorants, flavors, stabilizers, buffers, artificial and natural sweeteners, dispersants, thickeners, solubilizing agents, and the like.

Exemplary compositions for rectal administration include suppositories which can contain, for example, a suitable non-irritating excipient, such as cocoa butter, synthetic glyceride esters or polyethylene glycols, which are solid at ordinary temperatures, but liquefy and/or dissolve in the rectal cavity to release the drug.

Tasquinimod also may be administered parenterally, e.g. by injection or infusion, e.g. by intravenous, intraarterial, intraosseous, intramuscular, intracerebral, intracerebroventricular, intrasynovial, intrasternal, intrathecal, intralesional, intracranial, intratumoral, intracutaneous and subcutaneous injection or infusion. Thus, for parenteral administration, the pharmaceutical compositions may be in the form of a sterile injectable or infusible preparation, for example, as a sterile aqueous or oleaginous suspension. This suspension may be formulated according to techniques known in the art using suitable dispersing or wetting agents (e.g., Tween^{®} 80), and suspending agents. The sterile injectable or infusible preparation may also be a sterile injectable or infusible solution or suspension in a non-toxic parenterally acceptable diluent or solvent. For example, the pharmaceutical composition may be a solution in 1,3-butanediol. Other examples of acceptable vehicles and solvents that may be employed in the compositions of the present invention include, but are not limited to, mannitol, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically acceptable oils, such as olive oil or castor oil, especially in their polyoxyethylated versions. These oil solutions or suspensions may also contain a long-chain alcohol diluent or dispersant.

Solutions for parenteral use also may contain suitable stabilizing agents, and if necessary, buffer substances. Suitable stabilizing agents include antioxidizing agents, such as sodium bisulfate, sodium sulfite or ascorbic acid, either alone or combined, citric acid and its salts and sodium EDTA. Parenteral solutions may also contain preservatives, such as benzalkonium chloride, methyl- or propyl-paraben, and chlorobutanol.

Conventional procedures for the selection and preparation of suitable pharmaceutical formulations are described, for example, in "Pharmaceutics - The Science of Dosage Form Design", M.B. Aulton, Churchill Livingstone, 2nd ed. 2002 (ISBN 0443055173, 9780443055171). Suitable pharmaceutical excipients, e.g. carriers, and methods of preparing pharmaceutical dosage forms also are described in Remington's Pharmaceutical Sciences, Mack Publishing Company, a standard reference text in art of drug formulation.

The pharmaceutical compositions (which may also be referred to herein as pharmaceutical formulations, or medicaments) will comprise a therapeutically effective amount of tasquinimod or a pharmaceutically acceptable salt thereof, e.g. they may comprise from approximately 1 % to approximately 95%, preferably from approximately 20% to approximately 90% of tasquinimod or the salt thereof, together with at least one pharmaceutically acceptable excipient. In general, tasquinimod or its salt will be administered in a therapeutically effective amount by any of the accepted modes of administration for agents that serve similar utilities.

While e.g. injection or rectal administration of tasquinimod or its salt may be contemplated if necessary, oral administration generally is considered the most convenient.

The dosage level and frequency will generally be as determined by the treating physician, with due regard to factors such as and the sex, age, corporal weight and relative health of the treated subject, the severity of the MDS, the selected route and form of administration, the additional use of other drugs, e.g. in a combination therapy.

Generally, a daily dosage ranging from a minimum of 0.001 mg/kg body weight, or 0.002 mg/kg body weight or 0.005 mg/kg body weight or 0.01 mg/kg body weight, to a maximum of 0.2 mg/kg body weight, or 0.1 mg/kg body weight, or 0.05 mg/kg body weight, or 0.02 mg/kg body weight is contemplated.

In one embodiment, tasquinimod is administered in an amount of 0.05 to 0.15 mg/day, or 0.08 to 0.1 mg/day, e.g. 0.1 mg/day.

In one embodiment, tasquinimod is administered in an amount of 0.1 to 0.3 mg/day, or 0.15 to 0.25 mg/day, e.g. 0.2 mg/day.

In one embodiment, tasquinimod is administered in an amount of 0.1 to 1 mg/day, or 0.2 to 0.8 mg/day, e.g. 0.5 mg/day.

In one embodiment, tasquinimod is administered in an amount of 0.2 to 1.5 mg/day, or 0.4 to 1.2 mg/day, e.g. 0.8 mg/day.

In one embodiment, tasquinimod is administered in an amount of 0.5 to 2 mg/day, or 0.8 to 1.2 mg/day, e.g. 1 mg/day.

In one embodiment, tasquinimod is administered in an amount of 0.8 to 3 mg/day, or 1 to 2.5 mg/day, e.g. 2 mg/day.

In one embodiment, tasquinimod is administered in an amount of 1 to 6 mg/day, or 2 to 4 mg/day, e.g. 3 mg/day.

In some embodiments, the dosage may be gradually adjusted to reach optimal results, so-called dosage titration. For example, dosage titration may comprise starting with a low daily dosage of e.g. 0.25 mg and maintaining this dose level for a period of 1 or 2 weeks. In case no significant side effects are encountered that may contraindicate raising the dose, the level may then be increased, e.g. to 0.5 mg/day for 1 or 2 weeks, after which period another increase may be contemplated, to reach a daily dosage of 1 mg, and so on. In such a method, if any significant side effects occur after an incremental increase of the dosage, the dosage may again be reduced to a previous level.

Side effects that may occur include those that may generally be encountered in this type of treatment, e.g. gastrointestinal problems, tiredness, and flu-like syndrome, considered to be related to dosage.

Tasquinimod preferably is administrated on a daily basis, e.g. 1-3 times a day, or 1-2 times a day, such as once daily. In some embodiments, the drug is administrated on a less frequent basis, e.g. every two days, once a week etc. It should also be noted that if a pharmaceutically acceptable salt of tasquinimod is administered, an equivalent dosage would be one resulting in the indicated dosage of the compound in non-salt form.

In its broadest sense, the present invention relates to tasquinimod or a pharmaceutically acceptable salt thereof, for use in the treatment of MDS. In some embodiments, the MDS is selected from any of the subtypes mentioned herein above.

### BIOLOGICAL ASSAYS

### Materials and methods

### Patients

Heparinized BM samples were obtained from untreated MDS patients (low-risk MDS, low-risk MDS with del5q, high-risk MDS, and CMML) during standard diagnostic aspiration, and from hematological healthy donors undergoing hip replacement surgery. The samples were analyzed for level of S100A9 using the Human DuoSet ELISA kit (R&D Systems) according to manufacturer's instructions.

### Cell culture

BM mononuclear cells were isolated by percoll gradient centrifugation and MSCs were grown as adherent monolayer in DMEM/10% FCS. Passages 2-5 were used for the experiments.

To mimic inflammatory conditions, *in-vitro* cultured MSCs were treated with recombinant S100A8, S100A9 or the S100A8/9 heterodimer (each 1.5 µg/ml) or TNF-α (10 ng/ml) in the presence or absence of tasquinimod (10 µM) for different time periods depending on the subsequent experiments. Hematopoietic stem and progenitor cells (HSPCs) for cocultures were isolated using CD34 antibody-conjugated magnetic beads, according to the manufacturer's instructions (Miltenyi Biotec) and were added in CellGro medium (CellGenix) containing stem cell factor (SCF), FLT3-L and IL-3 (10 ng/ml each).

Peripheral blood mononuclear cells (PBMCs) were prepared by Ficoll-Hypaque density centrifugation.

### Clonogenic assays

CAF-C assays were performed over four weeks using pre-treated healthy or MDS MSC layers. One thousand magnetically isolated CD34+ cells were added in StemMACS^{™} HSC-CFU complete medium (Miltenyi Biotec).

CFU assays were carried out using cells harvested after one week of co-culture, with 300 cells being plated in enriched methylcellulose medium with recombinant cytokines (MethoCult^{™} H4435, STEMCELL Technologies). Colonies were counted after 2 weeks and classified under a microscope or with the STEMvision^{™} system (STEMCELL Technologies).

### MSC differentiation assays

Adipogenic and osteogenic differentiation capacity is an important functional feature of MSCs and often altered in MDS leading to changes in the bone metabolism. Therefore, the effect of tasquinimod on adipogenic and osteogenic differentiation was assayed as follows.

MSCs were seeded in 6-well plates (5 × 10³ MSCs/cm²), cultured to subconfluency for approximately 4 days in DMEM, and subjected to adipogenic (0.5 mM 1-methyl-3-butylisoxanthine, 1 µM dexamethasone, 100 µM indomethacin, 10 µM insulin) or osteogenic (0.1 µM dexamethasone, 0.2 mM ascorbate-2-phosphate, 10 mM β-glycerophosphate) differentiation. Adipogenesis was assessed by Oil Red O staining after 21 days. Differentiation was graded according to microscopic analysis as follows: 0 = no differentiation; 1 = 25% of the culture wells contain differentiated cells; 2 = 50% of the culture wells contain differentiated cells; 3 = 75% of the culture wells contain differentiated cells; 4 = 100% of the culture wells contain differentiated cells. Osteogenesis was determined by van Kossa staining, and the catalytic activity of ALP was determined.

### Detection of reactive oxygen species (ROS) levels

The inflammatory micromilieu of the myelodysplastic bone marrow leads to increased ROS levels which may cause aberrant cellular function. Therefore, the effect of tasquinimod on ROS levels in MSCs is assayed using the CellROX^{™} Deep Red Flow Cytometry Assay kit (Thermo Fisher) according to the manufacturer's instructions.

### Seahorse Metabolic Extracellular Flux Profiling

The impact of tasquinimod on cellular metabolism is investigated by using the Seahorse system (Agilent Technologies). MSCs are seeded in Seahorse 96-well plates. Cell Mito Stress Test (XF Cell Mito Stress Test Kit, Agilent Technologies) is performed following the standard protocol. Oxygen consumption rate (OCR) and Extracellular Acidification Rate (ECAR) are detected after injection of oligomycin (1 mM), carbonyl cyanide-p-trifluoromethoxyphenylhydrazone (FCCP, 0.5 mM), and the combination of rotenone and antimycin (Rot/AA, 0.5 mM). OCR is measured with an XF96 analyzer and the Wave software (version 2.2.0).

### Western blot

Whole cell lysates were prepared using RIPA lysis buffer containing proteinase inhibitors. Protein concentrations were determined using the BCA Protein Assay Kit. Equal protein amounts were separated by SDS-polyacrylamide gel electrophoresis and transferred to polyvinylidene fluoride membranes for detection of specific proteins using primary antibodies for IRAK1, NF-κB-p65, gasdermin and OXPHOS overnight at 4°C, following secondary antibodies HRP-conjugated goat anti-mouse IgG (Invitrogen) or donkey anti-rabbit IgG (GE Healthcare), respectively. The blots were incubated with ECL Plus Western Blotting reagent (Amersham) and the signals captured using a LAS3000 imaging system.

### RT-PCR

RNA was isolated from MSC using RNeasy Mini Kit (Qiagen) and reverse transcribed into cDNA using RevertAid^{™} cDNA synthesis kit (Thermo Fisher) with oligo-dT primers. Relative target quantity was determined using the comparative CT (ΔΔCT) method. RT-PCR was performed using SYBR Green/ROX PCR master mix (Thermo) and target specific primers for caspase1, IL1β, IL18, PD-L1, PD-1 and GAPDH as housekeeping gene on a Taqman^{™} Fast 3500 cycler (Applied Biosystems). Amplicons were normalized to endogenous GAPDH control.

### Immunohistochemistry

Bone marrow tissues of MDS patients and healthy donors were characterized by multiplex immunohistochemistry. With this method the sequence and spatial distribution of CD271+ MSCs, CD68+ macrophages and CD66b+ neutrophils were determined in one staining and analyzed with the VECTRA^{®} imaging system.

### Immunofluorescence staining and confocal laser scanning microscopy

S100A9/tasquninimod treated MSCs were fixed with 4% paraformaldehyde. Cells were permeabilized using PBS containing 0.1% Triton^{™} X-100 (T-PBS), blocked with T-PBS containing 10% FCS and 1% HSA (IF-Buffer) and incubated over night at 4°C with antibodies against αSMA, NFκB or PD-L1. Secondary antibodies polyclonal sheep-anti-rabbit-Cy3 or polyclonal goat-anti-mouse-Cy2 were incubated for 1 h at room temperature. Cell nuclei were counterstained with DAPI. Imaging analysis was performed by confocal microscopy (LSM800, Carl Zeiss) and ZEN software.

### Isolation and characterization of extracellular vesicles (EVs) from tasquinimod treated MSCs

EVs play an important role for the intercellular communication. These small membrane-surrounded particles carry different bioactive substances such as small RNA, mRNA and proteins. They are derived from the endosomal compartment or bud directly from the cell membrane. To test an influence of S100A9 and tasquinimod on MSC-derived EVs, they are isolated from serum-free culture supernatants by using the ExoEasy Maxi kit (Qiagen). Concentration and size of the EVs is detected by Nanoparticle Tracking Analysis (NTA) using a ZetaView^{®} instrument. The miRNA is isolated by the miRNeasy kit (Qiagen) and Taqman assays are performed with specific primers, e.g. for miR-145 and miR-146a.

The functional impact of S100A9/tasquinimod-primed MSC-derived EVs is analyzed by incubaton with PBMCs or HSPCs and subsequent flow cytometry.

### Results

Higher S100A9 levels were detected in BM plasma of MDS patients compared to healthy controls, with the highest levels measured in the low-risk MDS (Figure 1).

*In vitro,* relevant S100A9 mRNA expression could not be detected in cultured MDS or healthy MSCs, which suggests that this cell type does not represent the main S100A9 source in the bone marrow microenvironment. Rather, staining of BM tissue demonstrated S100A8 and S100A9 expression mainly by CD66b+ neutrophils and with less extent by CD68+ macrophages.

Treatment of MDS MSCs with S100A9 induced TLR4 downstream signalling, as demonstrated by increased expression of IRAK1 and NF-κB-p65. Additionally, higher expression of gasdermin was detected; gasdermin is an inductor of pyroptosis in S100A9 treated cells. Addition of tasquinimod inhibited the expression of the mentioned proteins, indicating an alleviation of the induced inflammation (Figure 2).

The mRNA expression of proinflammatory cytokines such as IL-1β and IL-18 as well as their activator caspase 1 increased in the MSCs after treatment with S100A9, and this increase was inhibited in the presence of tasquinimod (Figure 3).

PD-L1 as a potential downstream target that has the potential to inhibit efficient hematopoiesis in MDS, was induced by S100A9 and could be suppressed by tasquinimod both at the mRNA and protein level. It was noted that the basic expression of PD-L1 was clearly higher in CMML than in healthy MSCs (Figure 4). Surprisingly, the downregulation of PD-L1 was noted in MSC from healthy volunteers and CMML MDS patients alike when tasquinimod even in the absence of S100A9. This is surprising also since previously, tasquinimod has been reported to upregulate expression of PD-L1 (Oncoimmunology 2016, Vol. 5, No. 6, e1145333).

S100A9 treatment increased the expression of αSMA in MSCs which is typical for the differentiation of MSCs into myelofibroblasts present in the tumor microenvironment. In parallel, an increased nuclear expression of NF-κB was observed. Tasquinimod treatment reduces both αSMA and NF-kB expression.

Adipogenic differentiation is often pathologically increased in MDS MSCs. The addition of tasquinimod decreased the number of adipocytes by 25-50% after 14 days of cultivation in differentiation medium detected by Oil red O positive cells.

Addition of TNF-α to MSC cultures resulted in increased expression of IRAK1, NF-κB-p65 and gasdermin. Again, addition of tasquinimod inhibited the expression of these protein (Figure 5). This, too, indicates that the effect of tasquinimod in MSCs may not be related to S100A9.

Since functional alterations of MSCs induced by inflammasome activation have a significant impact on MDS progression, the potential of tasquinimod treatment of stromal cells to alter their subsequent support of normal hematopoiesis was investigated. To this end, MSC/HSPC *in vitro* co-cultures were used in which the stromal layers were pre-treated with combinations of S100A9 and tasquinimod. Clearly, treatment with S100A9 decreased the number of CAF-Cs as well as the number of CFUs in a subsequent clonogenic assay indicating a disturbed support of hematopoiesis by MSCs. Both the number of CAF-Cs and CFUs could be increased by tasquinimod (Figure 6a-c).

The PD-1 (receptor for PD-L1) expression in co-cultured HSPCs was regulated in the same way as its ligand in treated MSCs.

From the above findings it appears that the pathological inflammatory activation in the myelodysplastic bone marrow can be rescued by tasquinimod, by inhibition of NF-κB-p65 signalling and PD-L1/PD-1 expression in MSC. These effects result in an improved hematopoietic support by MSCs, and thereby to an improvement of anemia and cytopenia in MDS patients.

Finally, the results of the MSC differentiation assays show that tasquinimod is capable of reducing adipogenic differentiaton and of improving osteogenic differentiation. The results of the CellROX^{™} Deep Red Flow Cytometry Assay show that tasquinimod can block elevated ROS levels in MSCs. The results of the Seahorse Metabolic Extracellular Flux Profiling show that the effect of tasquinimod leads to reduced cellular stress and improved Spare Respiratory Capacity which is a readout for the cellular metabolic fitness.

### In vivo validation of in vitro results

The promising *in vitro* results are validated *in vivo* in different mouse models. In these models, genetically modified mice (e.g. B6;129-*Trp53bp1^{tm1Jc}*/J and NUP98/HOXD13) which develop typical signs of MDS such as anemia and cytopenia are treated with tasquinimod. Peripheral blood counts are recorded weekly and finally the bone marrow is analysed after 20 weeks. These experiments show that tasquinimod is able to partly restore functional hematopoiesis.

Moreover, immunodeficient NSG mice are transplanted with HSPCs which have been co-cultured with pre-treated MSCs as described above to analyse a potential improved engraftment of cells derived from tasquinimod pre-treated cocultures. Peripheral blood of the recipients are obtained every 3-4 weeks by venipuncture of the retro-orbital venous plexus and the amount of human cells is analysed by flow cytometry after staining with antibodies against human CD45 and CD34. The final bone marrow analysis is performed after 20 weeks.

## Claims

1. Tasquinimod, or a pharmaceutically acceptable salt thereof, for use in the treatment of myelodysplastic syndrome (MDS) in a mammal patient.

2. Tasquinimod, or a pharmaceutically acceptable salt thereof, for use according to claim 1, wherein the treatment is by oral administration.

3. Tasquinimod, or a pharmaceutically acceptable salt thereof, for use according to claim 1 or 2, wherein the treatment is by administration of an amount of from 0.001 mg to 0.2 mg of tasquinimod/kg of body weight per day, or of a corresponding amount of the pharmaceutically acceptable salt thereof.

4. Tasquinimod, or a pharmaceutically acceptable salt thereof, for use according to any one of claims 1 to 3, wherein the treatment is by administration of tasquinimod or the pharmaceutically acceptable salt thereof, 1-3 times a day.

5. Tasquinimod, or a pharmaceutically acceptable salt thereof, for use according to any one of claims 1 to 4, wherein tasquinimod or the pharmaceutically acceptable salt thereof is administered in a solid dosage form, such as a capsule, a tablet or a pill.

6. Tasquinimod, or a pharmaceutically acceptable salt thereof, for use according to any one of claims 1 to 4, wherein tasquinimod or a pharmaceutically acceptable salt thereof is administered dissolved or suspended in a liquid vehicle.

7. Tasquinimod, or a pharmaceutically acceptable salt thereof, for use according to any one of claims 1 to 6, wherein the treatment further comprises radiation therapy and/or autologous stem cell transplantation.

8. Tasquinimod, or a pharmaceutically acceptable salt thereof, for use according to any one of claims 1 to 7, wherein the MDS is selected from MDS with unilineage (single lineage) dysplasia, MDS with multilineage dysplasia, MDS with ring sideroblasts (MDS-RS), MDS associated with isolated del chromosome abnormality, such as MDS with isolated del(5q), MDS with excess blasts, and MDS, unclassifiable.

9. Tasquinimod, or a pharmaceutically acceptable salt thereof, for use according to claim 8, wherein the MDS is MDS with unilineage (single lineage) dysplasia.

10. Tasquinimod, or a pharmaceutically acceptable salt thereof, for use according to claim 8, wherein the MDS is MDS with multilineage dysplasia.

11. Tasquinimod, or a pharmaceutically acceptable salt thereof, for use according to claim 8, wherein the MDS is MDS with ring sideroblasts (MDS-RS).

12. Tasquinimod, or a pharmaceutically acceptable salt thereof, for use according to claim 8, wherein the MDS is MDS associated with isolated del chromosome abnormality.

13. Tasquinimod, or a pharmaceutically acceptable salt thereof, for use according to claim 8, wherein the MDS is MDS with excess blasts.

14. Tasquinimod, or a pharmaceutically acceptable salt thereof, for use according to claim 8, wherein the MDS is MDS, unclassifiable.

15. Tasquinimod, or a pharmaceutically acceptable salt thereof, for use according to any one of claims 1 to 6, wherein the MDS includes chronic myelomonocytic leukemia.

## Patentansprüche

1. Tasquinimod oder ein pharmazeutisch verträgliches Salz davon zur Verwendung bei der Behandlung des myelodysplastischen Syndroms (MDS) bei einem Säugetierpatienten.

2. Tasquinimod oder ein pharmazeutisch verträgliches Salz davon zur Verwendung gemäß Anspruch 1, wobei die Behandlung durch orale Verabreichung erfolgt.

3. Tasquinimod oder ein pharmazeutisch verträgliches Salz davon zur Verwendung gemäß Anspruch 1 oder 2, wobei die Behandlung durch Verabreichung einer Menge von 0,001 mg bis 0,2 mg Tasquinimod /kg Körpergewicht pro Tag oder einer entsprechenden Menge des pharmazeutisch verträglichen Salzes davon erfolgt.

4. Tasquinimod oder ein pharmazeutisch verträgliches Salz davon zur Verwendung gemäß einem der Ansprüche 1 bis 3, wobei die Behandlung durch Verabreichung von Tasquinimod oder des pharmazeutisch verträglichen Salzes davon 1 - 3-mal täglich erfolgt.

5. Tasquinimod oder ein pharmazeutisch verträgliches Salz davon zur Verwendung gemäß einem der Ansprüche 1 bis 4, wobei Tasquinimod oder das pharmazeutisch verträgliche Salz davon in einer festen Dosierungsform, wie zum Beispiel einer Kapsel, einer Tablette oder einer Pille, verabreicht wird.

6. Tasquinimod oder ein pharmazeutisch verträgliches Salz davon zur Verwendung gemäß einem der Ansprüche 1 bis 4, wobei Tasquinimod oder ein pharmazeutisch verträgliches Salz davon in einem flüssigen Träger gelöst oder suspendiert verabreicht wird.

7. Tasquinimod oder ein pharmazeutisch verträgliches Salz davon zur Verwendung gemäß einem der Ansprüche 1 bis 6, wobei die Behandlung ferner eine Strahlentherapie und/oder eine autologe Stammzelltransplantation umfasst.

8. Tasquinimod oder ein pharmazeutisch verträgliches Salz davon zur Verwendung gemäß einem der Ansprüche 1 bis 7, wobei das MDS ausgewählt ist aus MDS mit Einzellinien-Dysplasie (mit einzelner Abstammungslinie), MDS mit Mehrlinien-Dysplasie, MDS mit Ringsideroblasten (MDS-RS), MDS in Verbindung mit isolierter del-Chromosomenanomalie, wie beispielsweise MDS mit isolierter del (5q), MDS mit Blastenüberschuss und nicht klassifizierbarer MDS.

9. Tasquinimod oder ein pharmazeutisch verträgliches Salz davon zur Verwendung gemäß Anspruch 8, wobei es sich bei dem MDS um ein MDS mit Einzellinien-Dysplasie (mit einzelner Abstammungslinie) handelt.

10. Tasquinimod oder ein pharmazeutisch verträgliches Salz davon zur Verwendung gemäß Anspruch 8, wobei es sich bei dem MDS um ein MDS mit Mehrlinien-Dysplasie handelt.

11. Tasquinimod oder ein pharmazeutisch verträgliches Salz davon zur Verwendung gemäß Anspruch 8, wobei es sich bei dem MDS um ein MDS mit Ringsideroblasten (MDS-RS) handelt.

12. Tasquinimod oder ein pharmazeutisch verträgliches Salz davon zur Verwendung gemäß Anspruch 8, wobei es sich bei dem MDS um ein MDS in Verbindung mit isolierter del-Chromosomenanomalie handelt.

13. Tasquinimod oder ein pharmazeutisch verträgliches Salz davon zur Verwendung gemäß Anspruch 8, wobei es sich bei dem MDS um ein MDS mit Blastenüberschuss handelt.

14. Tasquinimod oder ein pharmazeutisch verträgliches Salz davon zur Verwendung gemäß Anspruch 8, wobei es sich bei dem MDS um ein nicht klassifizierbares MDS handelt.

15. Tasquinimod oder ein pharmazeutisch verträgliches Salz davon zur Verwendung gemäß einem der Ansprüche 1 bis 6, wobei das MDS eine chronische myelomonozytäre Leukämie einschliesst.

## Revendications

1. Tasquinimod, ou un sel pharmaceutiquement acceptable de celui-ci, pour son utilisation dans le traitement du syndrome myélodysplasique (SMD) chez un patient mammifère.

2. Tasquinimod, ou un sel pharmaceutiquement acceptable de celui-ci, pour son utilisation selon la revendication 1, dans laquelle le traitement est administré par voie orale.

3. Tasquinimod, ou un sel pharmaceutiquement acceptable de celui-ci, pour son utilisation selon la revendication 1 ou 2, dans laquelle le traitement consiste en l'administration d'une quantité comprise entre 0,001 mg et 0,2 mg de tasquinimod/kg de poids corporel par jour, ou d'une quantité correspondante du sel pharmaceutiquement acceptable de celui-ci.

4. Tasquinimod, ou un sel pharmaceutiquement acceptable de celui-ci, pour son utilisation selon l'une quelconque des revendications 1 à 3, dans laquelle le traitement consiste en l'administration de tasquinimod ou du sel pharmaceutiquement acceptable de celui-ci, 1 à 3 fois par jour.

5. Tasquinimod, ou un sel pharmaceutiquement acceptable de celui-ci, pour son utilisation selon l'une quelconque des revendications 1 à 4, dans laquelle le tasquinimod ou le sel pharmaceutiquement acceptable de celui-ci est administré sous une forme posologique solide, telle qu'une capsule, un comprimé ou une pilule.

6. Tasquinimod, ou un sel pharmaceutiquement acceptable de celui-ci, pour son utilisation selon l'une quelconque des revendications 1 à 4, dans laquelle le tasquinimod ou un sel pharmaceutiquement acceptable de celui-ci est administré dissous ou en suspension dans un véhicule liquide.

7. Tasquinimod, ou un sel pharmaceutiquement acceptable de celui-ci, pour son utilisation selon l'une quelconque des revendications 1 à 6, dans laquelle le traitement comprend en outre une radiothérapie et/ou une greffe de cellules souches autologues.

8. Tasquinimod, ou un sel pharmaceutiquement acceptable de celui-ci, pour son utilisation selon l'une quelconque des revendications 1 à 7, dans laquelle le SMD est choisi parmi le SMD avec dysplasie unilinéaire (lignée unique), le SMD avec dysplasie multilinéaire, le SMD avec sidéroblastes annulaires (SMD-RS), le SMD associé à une anomalie chromosomique de délétion isolée, tel que le SMD avec une del(5q) isolée, le SMD avec excès de blastes et le SMD non classifiable.oà

9. Tasquinimod, ou un sel pharmaceutiquement acceptable de celui-ci, pour son utilisation selon la revendication 8, dans laquelle le SMD est un SMD avec dysplasie unilinéaire (lignée unique).

10. Tasquinimod, ou un sel pharmaceutiquement acceptable de celui-ci, pour son utilisation selon la revendication 8, dans laquelle le SMD est un SMD avec dysplasie multilignée.

11. Tasquinimod, ou un sel pharmaceutiquement acceptable de celui-ci, pour son utilisation selon la revendication 8, dans laquelle le SMD est un SMD avec sidéroblastes annulaires (SMD-RS).

12. Tasquinimod, ou un sel pharmaceutiquement acceptable de celui-ci, pour son utilisation selon la revendication 8, dans laquelle le SMD est un SMD associé à une anomalie chromosomique de délétion isolée.

13. Tasquinimod, ou un sel pharmaceutiquement acceptable de celui-ci, pour son utilisation selon la revendication 8, dans laquelle le SMD est un SMD avec excès de blastes.

14. Tasquinimod, ou un sel pharmaceutiquement acceptable de celui-ci, pour son utilisation selon la revendication 8, dans laquelle le SMD est un SMD non classifiable.

15. Tasquinimod, ou un sel pharmaceutiquement acceptable de celui-ci, pour son utilisation selon l'une quelconque des revendications 1 à 6, dans laquelle le SMD comprend la leucémie myélomonocytaire chronique.
